# EUROPEAN PATENT APPLICATION

(11) **EP 1 055 399 A1**
(43) Date of publication of application: **29.11.2000**
(21) Application number: 00304244.7
(22) Date of filing: 19.05.2000
(51) Int. Cl.: A61B 18/12

(54) **An electrosurgical generator and system**

(30) Priority: 28.05.1999 GB 9912625
(71) Applicant: GYRUS MEDICAL LIMITED, Cardiff CF3 0LT (GB)
(72) Inventor: Penny, Keith, Upper Redbrook, Monmouth NP5 4LU (GB); Amoah, Francis Egyin, Whitchurch, Cardiff CF14 1TE (GB); Goble, Colin Charles Owen, Penarth, South Glamorgan CF64 1AT (GB)
(74) Representative: Blatchford, William Michael

(57) **Abstract**

An electrosurgical generator which is operable particularly at UHF or higher frequencies has a circuit (32) for sensing reflected power and a control arrangement (20) coupled to the sensing circuit to cause the frequency of the radio frequency output of the generator to be altered whilst the reflected power is monitored. The output frequency is then set to a value corresponding to a minimum reflected power level.

## Description

This invention relates to an electrosurgical generator and to an electrosurgical system incorporating an electrosurgical generator, and in particular to a generator and system in which electrosurgical power is delivered at a variable frequency.

The performance of an electrosurgical system having an electrode assembly coupled to an electrosurgical generator is related to the efficiency with which radio frequency power can be coupled into tissue being treated, and to the voltages and currents which can be generated at the distal tip of a treatment electrode.

The electrical load presented to the treatment electrode depends on a number of variables, including the frequency at which power is supplied to the electrode, the construction of the electrode, the mode of operation of the electrode, the nature of the tissue of other materials associated with the tissue being treated such as electrolytes, the orientation of the electrode during surgery, and the presence of other objects such as surgical instruments or nearby body structures. These factors cause load impedance to vary as a function of application and, in addition, as a function of time, not least because the electrical properties of the tissue being treated and those of neighbouring materials and objects change as a result of the surgery. In such circumstances of varying load impedance, efficiency varies widely. Efficiency is often judged as being maximised when the maximum power is delivered to the electrical load presented to the treatment electrode. Maximum power delivery occurs when the load presents a conjugate impedance match to the driving impedance of the electrosurgical system (i.e. the generator and electrode assembly).

As the frequency of operation is increased, and particularly when the generator is adapted to deliver electrosurgical power at UHF, maximum power delivery is dependent upon the generator frequency remaining close to a preferred frequency of operation. This preferred frequency or associated small frequency range may vary from electrode to electrode due to manufacturing tolerances and ageing effects due to storage and electrode use. These represent additional factors, therefore, affecting electrosurgical efficiency.

It is an objective of the present invention to improve the efficiency of an electrosurgical system.

Accordingly, one aspect of the invention is the optimisation of electrosurgical performance through automatic adjustment of the frequency of operation of the electrosurgical generator. This may be performed by analysing a reflected power signal in a control system which adjusts the frequency of a frequency source within the generator in order to optimise electrode performance. In particular, the invention provides an electrosurgical generator having a variable frequency radio frequency source, a reflected power sensing circuit and a control arrangement coupled to the sensing circuit and the source, wherein the control arrangement is adapted to vary the source frequency whilst receiving a reflected power output signal from the sensing means indicative of the reflected power, and to set the source frequency to a value corresponding to a relatively low reflected power level. Preferably, the frequency is adjusted to minimise the amplitude of the reflected power signal which, in general, correlates with maximum delivered electrosurgical power. Thus, in the preferred generator, the control arrangement is adapted to cause the source periodically to generate a plurality of different selected test frequencies and to monitor the said output signal at each test frequency, and subsequently to set the source to an operation frequency corresponding substantially to a minimum reflected power.

In this way, performance is optimised by automatic adjustment of the frequency of operation, thereby taking account of changes in load impedance due to the factors described above, and making it possible to relax the electrode manufacturing tolerances with a subsequent reduction in cost.

The sensing means may comprise a voltage or a current monitor associated with an output stage of the generator, or with the power output line between the output stage and the output terminal of the generator for connection to an electrode. Typically, the voltage or current monitor includes a hybrid coupler or circulator in the output line. Either or both of the amplitude and phase of the voltage or current produced by the sensing circuit constitute a measure of the impedance presented to the electrode coupled to the output terminal of the generator, and therefore such amplitude or phase constitutes a measure of power delivery efficiency.

Continuous or periodic monitoring of the return signal amplitude allows continuous or periodic adjustment of the source frequency for maximum power delivery. Indeed, the return signal may be continuously compared to a reference threshold as an indication of increased reflected power, triggering a frequency change for the source, whereupon the control arrangement monitors the new reflected power level to determine whether the frequency was adjusted in the correct direction for increasing efficiency.

Alternatively, the control arrangement may be adapted to perform a test sequence periodically, in which sequence the source frequency is varied over a predetermined range (which may be initially a maximum range, and subsequently a reduced range) and the said output signal is then monitored. In a subsequent treatment phase, the control arrangement is adapted to set the source to an operating frequency, which, on the basis of the monitored output signal during the test sequence, is selected to correspond to the frequency at which maximum delivered output power is obtained. The test sequence may be carried out at a repetition rate of at least twice every second and with a duty cycle of between 0.2 and 5%.

If it is anticipated that radio frequency interference may be troublesome, the source can be arranged to deliver electrosurgical power at a lower power setting during the test sequence than during the treatment phase.

In another embodiment, the frequency may be switched between different frequencies to determine the direction of frequency adjustment required for increased power delivery to the tissue. This process may be continued on an iterative basis until no further improvement is obtained, or until the delivered power once again reduces, in which case the direction of frequency change is reversed. It is possible to perform this process continuously such that when the frequency for maximum power delivery is achieved, the control arrangement operates so as to switch the source frequency repeatedly about the optimum frequency.

In yet a further embodiment, the generator may include a modulator coupled to the radio frequency source and arranged to modulate the source output signal such that it contains at least two different frequency components. In this instance, the generator may have a detector which forms part of the sensing circuit and is arranged to provide a reflected power output signal indicative of the relative magnitudes of the reflected power at the component frequencies, the control arrangement being adapted to monitor these magnitudes thereby to determine the direction of frequency adjustment required to increase the delivered electrosurgical power. The modulation is preferably amplitude modulation at a predetermined modulation frequency, the depth of modulation being selected so as to distribute power between the carrier (the central frequency of operation) and two sidebands respectively above and below the carrier frequency. At optimum electrosurgical efficiency, the reflected power levels of the two sidebands are approximately equal. At non-optimum frequencies, the reflected power amplitudes of the two sideband components are unequal and may be processed by the control arrangement to determine the direction in which the source frequency should be adjusted to improve efficiency.

The generator may include an input for receiving a radiation monitoring signal coupled, for example, to a radio receiving antenna to be placed near the patient being treated, typically within a few metres, as a means of monitoring radio interference. The control arrangement may be adapted, in this case, to process the monitoring signal and to adjust the source in response to a predetermined characteristic of the radiation monitoring signal, such as breaching of an interference power threshold. The control arrangement may then operate to adjust the frequency of the source in response to the predetermined characteristic so as to reduce the interference.

With regard to the source itself, this may be capable of being adjusted in frequency across a continuous or discrete range of frequencies or bands of frequencies.

The invention also provides an electrosurgical system including a generator as described above, coupled to an electrode assembly including at least one treatment electrode coupled to an output of the generator.

According to a method aspect of the invention, a method of operating an electrosurgical system comprises delivering electrosurgical radio frequency power from a radio frequency source at a plurality of frequencies, monitoring reflected power at the different frequencies, and adjusting the source frequency so as to set an operating frequency associated with a reflected power level which is lower than the reflected power levels associated with the other frequencies at which the source is operated.

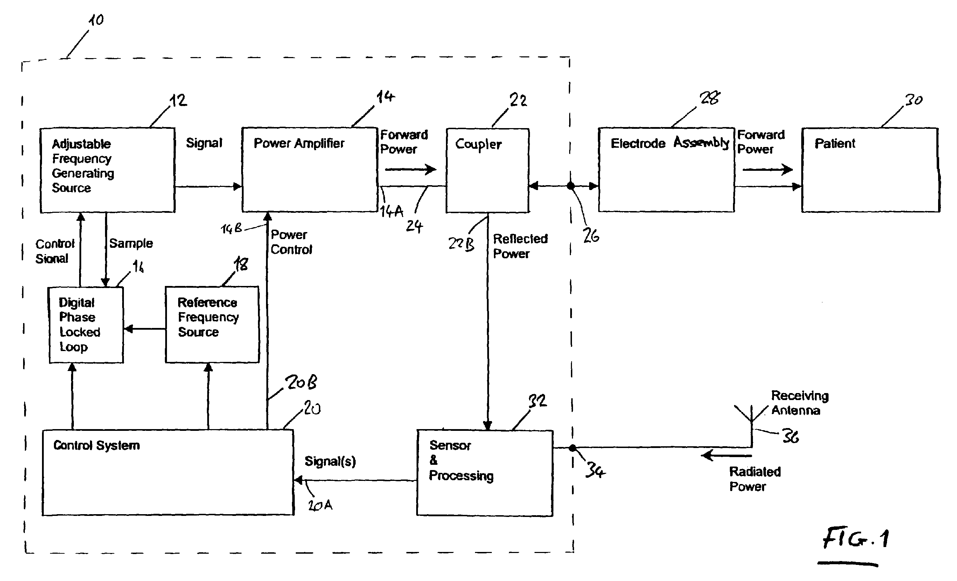

The invention will now be described by way of example with reference to the drawing in which Figure 1 is a block diagram showing an electrosurgical system including an electrosurgical generator in accordance with the present invention.

Referring to Figure 1, a generator 10 in accordance with the invention has a variable radio frequency source comprising an oscillator 12 and a power amplifier 14. Oscillator 12 forms part of the frequency synthesiser having a digital phase locked loop 16 coupled to the oscillator and arranged to receive a reference frequency signal from a reference frequency source 18 for comparison with a frequency-divided sample of the oscillator output. A control arrangement 20 is adapted to feed control signals to the phase locked loop (e.g. to alter its division ratio), and to the reference frequency source 18 (which also includes a frequency divider) for altering the frequency of the oscillator 12. The arrangement 20 also provides a power control output to the power amplifier 14 for adjusting the nominal output power of the generator.

It is possible to use analogue (rather than digital) control of the source frequency to achieve continuous and fine frequency adjustment through a negative feedback mechanism.

For example, oscillator 12 may be a voltage-controlled oscillator whereby the frequency is determined by a control voltage. This voltage may be generated by a current generating source feeding a capacitor. The control arrangement produces positive or negative current flow according to whether, respectively, an increase or decrease (or, depending on the design of the oscillator, a decrease or increase respectively) in operating frequency is required.

The preferred generator supplies electrosurgical power at UHF via a directional coupler 22 which is coupled in the output line 24 of the generator 10, extending between an output 14A of the source 12, 14 to a generator output terminal 26. In Figure 1, the output terminal 26 is shown connected to an electrode assembly 28 having a treatment electrode which, during use, is applied to the patient 30, and in particular to the tissue to be treated.

A circulator may be used in the coupler 22 to provide the power amplifier 14 with a nominal 50 ohm load under all conditions. A separate directional coupler may be used for reasons of optimisation, convenience and cost. The coupler 22 is connected in series between the radio frequency source 12, 14 and the electrode assembly 28, and reflected power is directed to a reflected power output 22B of the coupler which is connected to a sensing circuit 32 providing a nominal 50 ohm reflected power dump for the coupler 22.

As described above, unless a perfect conjugate impedance match exists between the generator 10 and the electrical load represented by the electrode assembly 28 and its surroundings (including patient 30), some of the power fed from the output terminal 26 of the generator to the electrode assembly is returned as reflected power. This is measured as a voltage and/or a current by the sensing circuit 32 which provides a reflected power output signal indicative of the magnitude of the reflected power to an input 20A of the control system 20.

The control system 20 makes use of the reflected power output signal from the sensing circuit 32 to determine an optimum frequency of operation. It will be appreciated that when the electrode is applied to the patient, the impedance conditions experienced by the generator 10 are largely unknown, which means that the generator characteristics, in particular its frequency, needed for best performance are not necessarily known in advance and vary during use.

The control system operates to vary the frequency of the oscillator 12 whilst monitoring the reflected power output signal of its input 20A to drive the frequency towards an optimum value at any given time during operation. In order to achieve this, the reflected power output signal may be continuously or periodically monitored. Similarly, the source frequency may be continuously or periodically adjusted.

In one mode the frequency of the source is adjusted by the control system across its maximum range and the frequency for maximum power delivery determined. Repetition of this process at a suitable duty cycle enables maximum power delivery. The process may be repeated to obtain e.g. 20 reflected power samples at different test frequencies separated by 5MHz intervals typically. This results in a 100 MHz test band. The 20 samples are typically taken in 2ms, once every half second. It is preferable, in order to reduce interference with other users or nearby equipment, momentarily to reduce the power output during the process of adjusting the frequency across its range via power control output 20B.

In another mode, the frequency of the source 12, 14 is switched between frequencies to determine the direction of frequency adjustment required for more power delivery to the tissue. This process may be continued until no further improvement is obtained or the power delivery is reduced in which case the direction of frequency change is reversed. Where the frequency for maximum power delivery is fixed, the control system will result in switching the operating frequency about the optimum frequency.

In another mode, amplitude modulation at a given modulation frequency and depth of modulation is applied to the radio frequency signal by the control system 20 or from a separate modulator (not shown). Power is therefore distributed according to the depth of modulation between the radio frequency carrier (at the frequency of operation) and two sidebands (at an offset above and below the carrier frequency equal to the modulating frequency). The two sidebands contain equal (or roughly equal where the power output of the equipment varies with frequency) amounts of power. Measurement of the reverse power signal (using a single sideband detector in the sensing circuit 32) enables any significant imbalance between the two sidebands to be detected so that the control system 20 can determine the direction in which the frequency of operation is to be adjusted.

In yet another mode, the source 12, 14 is operated in a test phase during which it produces several different frequency components simultaneously using, for instance, spread-spectrum modulation, and the sensing circuit 32 samples at particular frequencies or relatively narrow bands of frequencies to determine the best operating frequency for normal operation during the operation phase.

In order to reduce or prevent radio frequency interference with other radio frequency users or with equipment in the vicinity, it is possible to adjust the frequency of operation to reduce radiated interference or to exclude particular frequencies or bands or frequencies from use. In this instance, the control system is configured to alter the frequency of the source 12, 14 by monitoring an additional signal from the sensing circuit 32 which is generated in response to a radiation monitoring signal antenna 36. This antenna may be placed near the patient, typically within a few metres, the sensing circuit 32 including a receiver which is arranged to receive signals within the operating band of the generator 10 or in other bands (for instance, at a harmonic of the operating frequency). The control system 20 may monitor the radiation monitoring signal applied to its input 20A, which is representative of received signal power from antenna 36, which is processed or to cause a reduction in nominal output power via power control input 14B of the power amplifier 14.

The same power control input 14B may be used to reduce the nominal radio frequency power output automatically during test phases, i.e. test phases in which the source frequency is varied to sample reflective power and/or radiated power via the sensing circuit 32.

## Claims

1. An electrosurgical generator having a radio frequency source, a reflected power sensing circuit and a control arrangement coupled to the sensing circuit and the source, wherein the control arrangement is adapted cause the source to operate at different source frequencies whilst receiving a reflected power output signal from the sensing means indicative of the reflected power, and to set the source frequency to a value corresponding to a relatively low reflected power level.

2. A generator according to claim 1, wherein the control arrangement is adapted to cause the source periodically to generate a plurality of different selected test frequencies and to monitor the said output signal at each test frequency, and subsequently to set the source to an operation frequency corresponding substantially to a minimum reflected power.

3. A generator according to claim 1 or claim 2, wherein the sensing means comprises a voltage or current monitor associated with a generator power output line.

4. A generator according to claim 3, wherein the monitor includes a hybrid coupler in the said output line.

5. A generator according to claim 1, wherein the control arrangement is adapted to monitor the output signal from the sensing means continuously during operation of the generator and to adjust the set frequency in response to the said output signal reaching a threshold value indicative of increased reflected power.

6. A generator according to claim 1, wherein the control arrangement is adapted to perform a test sequence periodically, in which sequence the source frequency is varied over a predetermined range and the said output signal is monitored, and then, in a treatment phase, to set the source to an operating frequency selected in response to the output indicative of a maximum delivered output power.

7. A generator according to claim 6, in which the control arrangement is adapted to perform the test sequence at least every 0.5 seconds.

8. A generator according to claim 6 or claim 7, wherein the source is arranged to deliver electrosurgical power at a lower power setting during the test sequence than in the treatment phase.

9. A generator according to claim 1, wherein the control arrangement is adapted to cause the source to switch between different source frequencies and to monitor the said output signal to determine the direction of frequency adjustment required to increase the delivered electrosurgical power.

10. A generator according to claim 9, wherein the control arrangement is adapted to cause the said switching repeatedly and progressively to adjust the source frequency in an interactive manner to a frequency substantially corresponding to maximum power.

11. A generator according to claim 1, including a modulator coupled to the source and arranged to modulate the source output signal such that it contains at least two different frequency components, and a detector forming part of the sensing circuit and arranged to provide a reflected power output signal indicative of the magnitudes of the reflected power at the frequencies of the said components, and wherein the control arrangement is adapted to monitor the relative magnitudes of the reflected power corresponding to the different frequency components and to adjust the source frequency to a value tending to increase the delivered electrosurgical power.

12. A generator according to claim 11, wherein the detector is a single sideband detector.

13. A generator according to claim 1, wherein the control arrangement and/or the source are adapted to generate, during a test phase, a source output signal having, simultaneously, a plurality of different frequency components, and wherein the sensing circuit is operable to sample a reflected power signal at the frequencies of said components, the control arrangement being adapted to process the values of the resulting sensor output signals to select an operating frequency.

14. A generator according to claim 12, wherein the frequency components are generated by spread-spectrum modulation.

15. A generator according to any preceding claim, including an input for receiving a radiation monitoring signal, the control arrangement being adapted to process the monitoring signal and to adjust the source in response to a predetermined characteristic of the radiation monitoring signal.

16. A generator according to claim 15, wherein the control arrangement is adapted to adjust the frequency of operation of the source in response to the said predetermined characteristic.

17. An electrosurgical system including a generator according to any preceding claim, and an electrode assembly including at least one treatment electrode coupled to an output of the generator.

18. A system according to claim 17, including a generator according to claim 15 or claim 16 and an antenna coupled to the said input.
